# EUROPÄISCHE PATENTSCHRIFT

(11) **EP 1 527 097 B1**
(45) Veröffentlichungstag und Bekanntmachung des Hinweises auf die Patenterteilung: **23.01.2008**
(21) Anmeldenummer: 03766211.1
(22) Anmeldetag: 18.07.2003
(51) Int. Cl.: C07K 14/62

(54) **VERFAHREN ZUR REINIGUNG VON PREPROINSULIN**
METHOD FOR PURIFYING PREPROINSULIN
PROCEDE POUR PURIFIER DE LA PREPROINSULINE

(30) Priorität: 01.08.2002 DE 10235168
(43) Veröffentlichungstag der Anmeldung: 04.05.2005
(73) Patentinhaber: Sanofi-Aventis Deutschland GmbH, 65929 Frankfurt am Main (DE)
(72) Erfinder: THUROW, Horst, 65582 Hambach (DE); BLUMENSTOCK, Hans, 65795 Hattersheim (DE); HAVENITH, Chantalle, 65719 Hofheim (DE)
(86) Internationale Anmeldenummer: PCT/EP2003/007820
(87) Internationale Veröffentlichungsnummer: WO 2004/013176

(56) Entgegenhaltungen:
- WO-A-01/25278
- WO-A-91/03550
- WO-A-99/33988
- US-A- 5 101 013
- COWLEY D J ET AL: "Expression, purification and characterization of recombinant human proinsulin." FEBS LETTERS. NETHERLANDS 3 FEB 1997, Bd. 402, Nr. 2-3, 3. Februar 1997 (1997-02-03), Seiten 124-130, XP002257683 ISSN: 0014-5793
- LADISCH M R ET AL: "Recombinant human insulin." BIOTECHNOLOGY PROGRESS. UNITED STATES 1992 NOV-DEC, Bd. 8, Nr. 6, November 1992 (1992-11), Seiten 469-478, XP001155556 ISSN: 8756-7938

## Beschreibung

Weltweit leiden etwa 12 Mio. Menschen an dem Typ 1 des Diabetes mellitus, der durch eine unzureichende körpereigene Produktion des Hormons Insulin gekennzeichnet ist. Für diesen Typus des Diabetes mellitus stellt die Substitution der fehlenden endokrinen Insulinsekretion durch Applikation von Insulinpräparaten die einzig mögliche Therapieform dar.

Insulinpräparate sind pharmazeutische Zubereitungen, die als Wirkstoff das Hormon Insulin enthalten. Dabei werden nicht nur natürlich vorkommende Insuline verwendet, sondern auch Insulinanaloga und Insulinderivate.

Das in der menschlichen Bauchspeicheldrüse produzierte Humaninsulin ist ein Polypeptid bestehend aus 51 Aminosäureresten, die sich auf 2 Peptidketten verteilen:
die A-Kette mit 21 Aminosäureresten und die B-Kette mit 30 Aminosäureresten. In beiden Peptidketten ist die Sequenz der Aminosäurereste genetisch determiniert und bekannt. Beide Ketten sind durch zwei Disulfidbrücken miteinander verbunden. Daneben befindet sich in der A-Kette noch eine intrachainare Disulfidbrücke.

Insulinanaloga unterscheiden sich vom Humaninsulin durch Substitution wenigstens eines Aminosäurerestes und/oder Addition oder Entfernen wenigstens eines Aminosäurerestes. Insulinanaloga können entweder in anderen Spezies als dem Menschen natürlich vorkommen oder sie können künstlich hergestellt worden sein. Insulinderivate enthalten chemisch modifizierte Aminosäurereste, die z.B. zusätzliche Ester- oder Amidogrupppen enthalten, ansonsten aber die humane oder eine analoge Aminosäuresequenz zeigen.

In der Regel zeigen Insulinanaloga bzw. Insulinderivate gegenüber dem unmodifizierten Humaninsulin eine veränderte Wirkungskinetik.

Seit einigen Jahren werden das Humaninsulin und die Insulinanaloga bzw. Insulinderivate durch die rekombinante DNA Technologie hergestellt. In industriellen Verfahren wird zum Beispiel zuerst ein entsprechender precursor der Formel 1, das Preproinsulin (PPI) hergestellt, aus dem durch enzymatische Spaltung das Humaninsulin oder die Insulinanaloga hergestellt werden. Zum Beispiel besteht ein gentechnologisches Verfahren zur Herstellung von Humaninsulin aus folgenden Verfahrensschritten:
a) Fermentation der gentechnisch veränderten Mikroorganismen
b) Ernten der Mikrooganismen und Zellaufschluß
c) Isolierung der Einschlußkörper mit dem ungelösten Fusionsprotein
d) Auflösen des Fusionproteins mit korrekter Faltung der Peptidkette und mit gleichzeitiger Schließung der Disulfidbrücken zum Preproinsulin
e) Enzymatische Spaltung des Preproinsulins zum Humaninsulin
f) Reinigung des Humaninsulins
g) Kristallisation des Humaninsulins und Trocknung des Abgabeprodukts

Bei der Herstellung eines Insulinanalogons ist die Aminosäuresequenz (der A- und B-Kette) in den entsprechenden Bereichen des Preproinsulins bereits vorgegeben. Für die enzymatische Spaltung der verschiedenen Preproinsuline werden Proteasen, wie zum Beispiel das Enzym Trypsin und falls notwendig zusätzlich das Enzym Carboxypeptidase B verwendet.

Das Preproinsulin ist ein Protein der Formel 1,

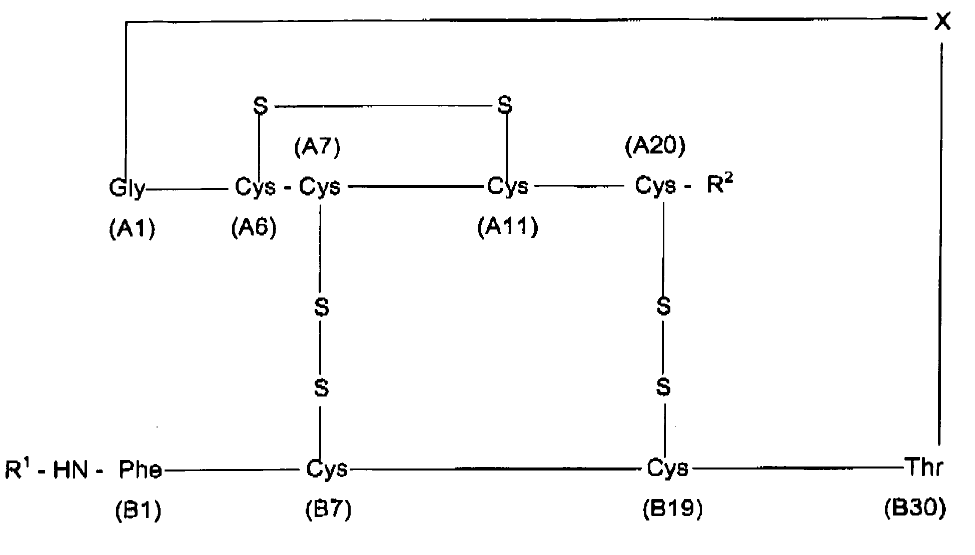

R¹-B1-B30-X-A1-A20-R² (1)

in der
- X: a) ein genetisch kodierbarer Aminosäurerest oder
b) ein Peptid mit 2 bis 35 Aminosäureresten, wobei am Anfang und am Ende des Peptids jeweils ein basischer Aminosäurerest, insbesondere Arg steht, und für den Fall, dass das Peptid aus mehr als 3 Aminosäureresten besteht, am Anfang und Ende des Peptids jeweils zwei basische Aminosäurereste, insbesondere Arg und/oder Lys stehen,
- R¹: a) ein Wasserstoffatom.
b) ein genetisch kodierbarer Aminosäurerest oder
c) ein Peptid mit 2 bis 15 Aminosäureresten,
- R²: ein genetisch kodierbarer Aminosäurerest ist und
und die Reste A1 - A20 der Aminosäuresequenz der A-Kette von Humaninsulin oder einem Insulinanalogon entsprechen und die Reste B1 - B30 der Aminosäuresequenz der B-Kette von Humaninsulin oder einem Insulinanalogon entsprechen.

Das Preproinsulin ist bevorzugt ein Protein der Formel 1, in der
- X: ein Peptid mit 35 Aminosäureresten mit der Sequenz der C-Kette von Humaninsulin oder Affeninsulin, oder ein Peptid mit 29 Aminosäuren mit der Sequenz:
- R¹: ein Peptid mit 2 bis 15 Aminosäureresten, an dessen Carboxylende der Aminosäurerest Arg steht,
- R²: der Aminosäurerest Asn oder Gly steht,
und die Reste A1 - A20 der Aminosäuresequenz der A-Kette von Humaninsulin entsprechen und die Reste B1 - B30 der Aminosäuresequenz der B-Kette von Humaninsulin oder einem Insulinanalogon entsprechen, bei dem in der B3-Position Lys anstatt Asn und in der B29-Position Glu anstatt Lys stehen.

In der Prozessstufe - Auflösen des Fusionsproteins mit korrekter Faltung der Peptidkette und mit gleichzeitiger Schließung der Disulfidbrücken zum Preproinsulin - entstehen neben dem erwünschten monomeren Preproinsulin in einer Konkurrenzreaktion auch polymere Formen des Preproinsulins. Diese polymeren Preproinsuline lassen sich aufgrund ihres höheren Molekulargewichtes durch die HPLC-GPC-Analyse oder durch die Methode der dynamischen Lichtstreuung nachweisen. Um diese unerwünschte Konkurrenzreaktion zurückzudrängen muss die Anfangskonzentration des Fusionsproteins möglichst gering sein (De Bernadez et al., Meth. Enzym. 309:217, 1999). In der Praxis entsteht in dieser Prozessstufe das Preproinsulin in einer Konzentration von ca. 0,5 bis 1 g/L und zusätzlich findet man ca. 40 % höhermolekulare Anteile. Unter den höhermolekularen Anteilen befinden sich die polymeren Preproinsuline.
Es wurde im Rahmen dieser Erfindung überraschenderweise gefunden, dass die polymeren Formen der Preproinsuline die Stabilität der Insuline auf den nachfolgenden Prozessstufen negativ beeinflussen, indem sie die Denaturierung der nativen Insuline induzieren. Es ist bekannt, dass während der Reaktionskette der Denaturierung in einem ersten reversiblen Schritt aus den gelösten monomeren Insulinmolekülen lineare Aggregate entstehen, in denen die Wiederholungseinheiten durch physikalische Adhäsionskräfte zusammengehalten werden. In einer irreversiblen Folgereaktion entstehen aus den gelösten Aggregaten stabile unlösliche Aggregatbündel (Fibrillen), die ihrerseits in einem autokatalytischen Prozess die Denaturierung nativer Insuline induzieren. Diese unlöslichen Insulinfibrillen sind nicht nur biologisch inaktiv, sondern sie können auch zum Verstopfen von Injektionskanülen bei der Applikation der pharmazeutischen Insulinpräparate führen. Daneben werden sie auch für immunologische Unverträglichkeitsreaktionen verantwortlich gemacht, die gelegentlich während der Therapie mit Insulinpräparaten auftreten können (J.Brange et al, J. Pharm. Sc. 1997, 86, 517-525; R.E. Ratner et al., Diabetes, 39, 728-733, 1990).

In der weiteren Folge des Insulin-Herstellungsprozesses wird das Preproinsulin mit Hilfe der Enzyme Trypsin und Carboxypeptidase B in Humaninsulin umgewandelt (siehe Kemmler, W., Peterson, J.D., and Steiner, D.F., J. Biol. Chem., 246 (1971) 6786-6791). Dabei werden das Verbindungspeptid zwischen der A- und B-Kette (X in der Formel 1) und der Preteil am Aminoende der B-Kette (R¹ in der Formel 1) abgespalten. Bei der enzymatischen Reaktion mit Trypsin werden nicht nur diejenigen Peptidbindungen gespalten, deren Spaltung zum Humaninsulin führt, sondern in einer konkurrierenden Reaktion auch andere Peptidbindungen, deren Spaltung zu mehreren unerwünschten Nebenprodukten führt. Besonders unerwünscht ist die Bildung von des-Thr-Insulin durch zusätzliche Spaltung zwischen den Aminosäureresten B29 und B30 in Formel 1 (siehe EP 0 264 250 B1). Die Abtrennung dieses Nebenproduktes in den nachfolgenden Reinigungsstufen führt zu großen Produktverlusten. Um diese unerwünschte Nebenreaktion zurückzudrängen, muss die Anfangskonzentration an Preproinsulin möglichst hoch sein, d.h. im Bereich von 8 - 25 g/L, entsprechend 1 - 3 mM (siehe EP 0 264 250 B1). Diese Forderung steht im Gegensatz zu der im vorvorgehenden Absatz erwähnten Forderung.
Aus dem oben gesagten geht hervor, daß es von Vorteil ist, zwischen der Herstellung des Preproinsulins und der Spaltung des Preproinsulins zum Insulin einen zusätzlichen Prozessschritt einzuführen, der die polymeren Preproinsuline möglichst vollständig entfernt und gleichzeitig die Konzentration des monomeren Preproinsulins möglichst stark erhöht. Dass bei diesem Prozessschritt eine möglichst hohe Stufenausbeute gewährleistet sein muss, ist eine zusätzliche Bedingung.

Es wurde deshalb vorgeschlagen (EP 0 600 372 B1), das Preproinsulin an ein hydrophobes Adsorberharz zu konzentrieren. Der Antragsteller konnte mit eigenen Versuchen zeigen, dass dabei zwar ein hoher Konzentrierungsfaktor von F = 10 - 15 erreicht werden kann, aber praktisch keine Abtrennung der polymeren Preproinsuline erfolgt. Ein weiterer Vorschlag (D.F. Steiner et al., Diabetes, 17 (1968), 725-736) erwähnt die chromatographische Reinigung des Preproinsulins mit Hilfe eines lonenaustauscherharzes. In eigenen Versuchen konnte mit einem Anionenaustauscherharz nur ein Konzentrierungsfaktor von F = 5 und eine Abtrennung der höhermolekularen Anteile bis auf ca. 5 % erreicht werden. Mit einem Kationenaustauscherharz wurden zwar die höhermolekularen Anteile bis auf ca. 1 % abgetrennt, aber die Bindungskapazität des Harzes für das Preproinsulin erwies sich als unbefriedigend.

In der internationalen Patentanmeldung WO99/33988 wird ein Verfahren zur Herstellung von humanem Proinsulin beschrieben, welches zunächst in Form von Einschlusskörpern exprimiert wird und dann durch Auflösung dieser Einschlusskörper, Sulfonierung, Reinigungsschritte, Konzentrierung und Entsalzung gekennzeichnet ist.

Im US-Patent 5,101,013 wird ein Verfahren zur Reinigung von basischen Proteinen beschrieben, die durch enzymatische Behandlung von Proinsulin und/oder seiner Derivate erhalten wurden, wobei insbesondere eine stark saure Kationenaustausch-Chromatographie verwendet wird.

In der internationalen Patentanmeldung WO91/03550 wird die Herstellung von Fusionsproteinen unter Verwendung eines "Ballastanteils" beschrieben, wobei ein vorteilhafter Ballastanteil zur Maximierung der Expressionsrate durch einen Versuchsansatz ermittelt wird, bei dem eine Mischung von verschiedenen Oligonukleotiden, welche für Ballastanteile kodieren, verwendet wird und schließlich diejenigen Expressionsplasmide ausgewählt werden, die für Ballastanteile kodierende Oligonukleotide enthalten, die hohe Expressionsraten ermöglichen.

Es wurde nun überraschend gefunden, dass die Kombination von einer Chromatographie an einem Anionenaustauscherharz im Durchflußmodus mit einer direkt anschließenden Chromatographie an einem Kationenaustauscherharz Im Adsorptionsmodus deutlich bessere Resultate lieferte. Die vorliegende Erfindung bezieht sich deshalb auf ein Verfahren zur effektiven Abtrennung der höhermolekularen Substanzen aus einer wässrigen Lösung des Preproinsulins bei gleichzeitiger hoher Konzentrierung des monomeren Preproinsulins.

Erfindungsgemäß wird eine verdünnte wässrige Lösung eines Preproinsulins, wie sie während des Herstellungsprozesses von Insulin entsteht, bei pH 7,0 bis 9,0 , vorzugsweise bei pH 7,5 bis 8,5 und einer Leitfähigkeit von 5 bis 7 mS/cm über eine Vorsäule gepumpt, die mit einem Anionenaustauscherharz, z.B. Source 30 Q, gefüllt ist. Dabei wird das monomere Preproinsulin nicht an das Harz gebunden, sondern läuft mit dem Permeat durch die Säule. Im Gegensatz dazu wird der größte Teil der höhermolekularen Substanzen, inklusive der polymeren Preproinsuline, an das Harz adsorbiert und damit vom Preproinsulin abgetrennt. Das Wertstoff-haltige Permeat dieser Vorsäule wird in line mit Salzsäure auf pH 3,0 bis 5,5 , vorzugsweise auf pH 4,0 bis 5,0 eingestellt und anschließend direkt auf eine zweite Säule gepumpt, die mit einem Kationenaustauscherharz, z.B. Source 30 S, gefüllt ist. An dieses Harz wird das Preproinsulin adsorbiert und Verunreinigungen werden mit dem Permeat aus der Säule gespült. Mit Hilfe eines Elutionspuffers, der Natriumchlorid mit einer linear ansteigenden Konzentration von 1 bis 20 g/L, vorzugsweise 2,5 bis 15,0 g/L enthält, wird das Preproinsulin desorbiert. Das gereinigte Preproinsulin wird in einer Hauptfraktion aufgefangen, wogegen weitere Verunreinigungen in einer Vor- und Nachfraktion abgetrennt werden. In der Hauptfraktion, die > 90 % der Ausgangsmenge an Preproinsulin enthielt, wurde eine Konzentration von 15 bis 20 g/L gemessen (Konzentrierungsfaktor F = 20 - 25). Die höhermolekularen Substanzen wurden bis auf einen Anteil von < 0,1 % abgetrennt. Das auf diese Weise gereinigte Preproinsulin kann durch Kristallisation aus der Lösung zwischenisoliert werden oder die Lösung kann direkt der Prozessstufe der enzymatischen Spaltung zugeführt werden.

Gegenstand der Erfindung ist somit ein Verfahren zur chromatographischen Reinigung von Preproinsulin der Formel 1,

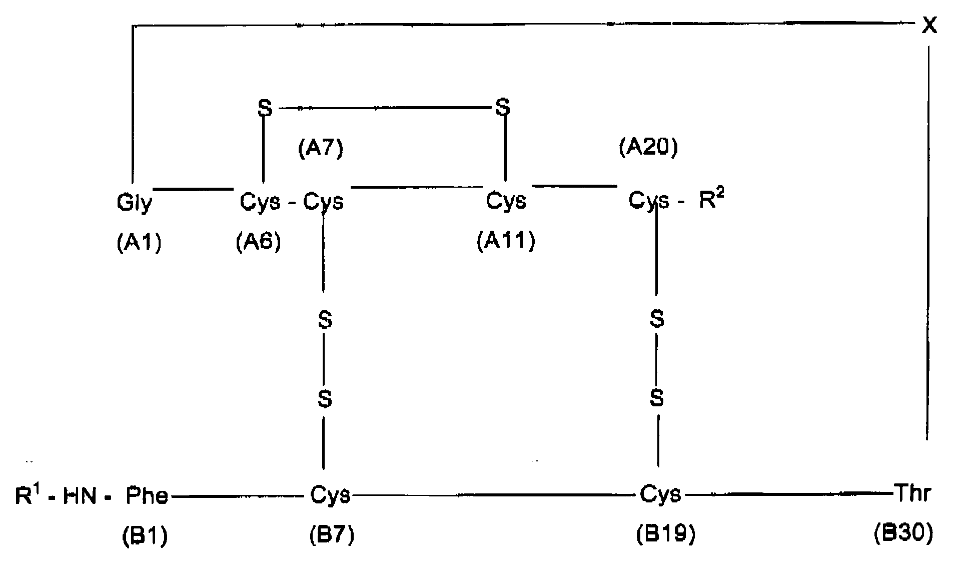

R¹-B¹-B30-X-A1-A20-R² (1)

in der
- X: a) ein genetisch kodierbarer Aminosäurerest oder
b) ein Peptid mit 2 bis 35 Aminosäureresten, wobei am Anfang und am Ende des Peptids jeweils ein basischer Aminosäurerest, Insbesondere Arg steht, und für den Fall, dass das Peptid aus mehr als 3 Aminosäureresten besteht, am Anfang und Ende des Peptids jeweils zwei basische Aminosäurereste, insbesondere Arg und/oder Lys stehen,
- R¹: a) ein Wasserstoffatom,
b) ein genetisch kodierbarer Aminosäurerest oder
c) ein Peptid mit 2 bis 15 Aminosäureresten,
- R²: ein genetisch kodierbarer Aminosäurerest ist und
und die Reste A1 - A20 der Aminosäuresequenz der A-Kette von Humaninsulin oder einem Insulinanalogon entsprechen und die Reste B1 - B30 der Aminosäuresequenz der B-Kette von Humaninsulin oder einem Insulinanalogon entsprechen;
bei dem höhermolekulare Substanzen aus einer wäßrigen Lösung des Preproinsulins durch eine erste Chromatographie an einem Anionenaustauscher im Durchflussmodus und eine anschließende zweite Chromatographie an einem Kationenaustauscher im Adsorptionsmodus abgetrennt werden;
wobei das Preproinsulin folgende Aminosäuresequenz haben kann:

Ein weiterer Gegenstand der Erfindung ist ein Verfahren wie oben beschrieben zur Abtrennung von Fremdsubstanzen aus den Lösungen von Preproinsulinen, welche die Denaturierung von Insulin induzieren.

Ein weiterer Gegenstand der Erfindung ist ein Verfahren wie oben beschrieben, dadurch gekennzeichnet, dass die zweite Chromatographie bei einem pH-Wert von 3,0 bis 5,5 durchgeführt wird.

Ein weiterer Gegenstand der Erfindung ist ein Verfahren wie oben beschrieben, dadurch gekennzeichnet, dass die zweite Chromatographie bei einem Druck von 1 bis 30 bar durchgeführt wird.

Ein weiterer Gegenstand der Erfindung ist ein Verfahren zur Herstellung von Insulin durch Expression ungefalteten Preproinsulins, umfassend die Schritte:
a) Fermentation von gentechnisch veränderten Mikroorganismen, welche ungefaltetes Preproinsulin exprimieren
b) Ernten der Mikrooganismen und Zellaufschluß
c) Isolierung der Einschlußkörper mit ungelöstem, ungefalteten Preproinsulin
d) Auflösen des Preproinsulins mit korrekter Faltung der Peptidkette und mit gleichzeitiger.Schließung der Disulfidbrücken zum Preproinsulin und anschließendem Durchlaufen eines Verfahren zur chromatographischen Reinigung von Preproinsulin der Formel 1 wie oben beschrieben
e) Enzymatische Spaltung des Preproinsulins zum Humaninsulin
f) Reinigung des Humaninsulins
g) Kristallisation des Humaninsulins und Trocknung

Im folgenden wird die Erfindung anhand der Beispiele näher erläutert, ohne sich darauf zu beschränken.

### Beispiele:

Für die in den nachfolgenden Beispielen 1 bis 3 beschriebene Reinigung verschiedener Preproinsuline der Formel 1 wurde die Ausgangslösung in bekannter Weise (EP 0 489 780 und EP 0 600 372) entsprechend den oben erwähnten Prozessstufen a, b, c und d wie folgt hergestellt:

Während der Fermentation der Mikroorganismen (Prozessstufe a) wurden in den E.-Coli-Zellen Einschlußkörper gebildet, die das Fusionsprotein mit der Aminosäuresequenz des Preproinsulins enthielten. Nach Beendigung der Fermentation wurden die Zellen durch Zentrifugation isoliert und durch die übliche Hochdruckhomogenisation aufgeschlossen (Prozessstufe b). Die dabei freigesetzten unlöslichen Einschlußkörper wurden durch Zentrifugation isoliert und auf der Zentrifuge mit Wasser gewaschen (Prozessstufe c). In der nachfolgenden Prozessstufe d wurden die Fusionsprotein-Einschlusskörper in einer 8 M Guanidinhydrochlorid-Lösung bei pH 10,8 gelöst. Nach Verdünnen mit Wasser und Zugabe von Cysteinhydrochlorid wurde das Fusionsprotein bei pH 10,8 und 4 °C unter Schließung der 3 Disulfidbrücken zu dem Preproinsulin der Formel 1 gefaltet. Anschließend wurde die Lösung mit 10 %iger Salzsäure auf pH 5 eingestellt und dabei Fremdproteine ausgefällt, die durch Zentrifugation abgetrennt wurden. Der Zentrifugenüberstand enthielt 0,6 bis 0,8 g/L monomeres Preproinsulin. Die Reinheit des Preproinsulins wurde mit der HPLC-RP-Analyse zu ca. 65 Flächen-% bestimmt. Mit der HPLC-GPC-Analyse wurde ein Anteil von ca. 45 Flächen-% höhermolekulare Verunreinigungen ermittelt. HPLC-RP-Analyse

| | |
|---|---|
| Säule: | LiChroCART 250-4 der Fa. Merck (Superspher 100-RP18e) |
| Instrument: | Waters 2690 |
| Software: | Waters Millenium |
| Gradient: | A: 25 Vol-% Acetonitril, 0,3 M NaCl in 0,05 M Phosphatpuffer pH 2,5 |
| | B: 65 Vol% Acetonitril, 0,05 M NaCI in 0,05 M Phosphatpuffer pH 2,5 |
| | Der Gradient ist gekennzeichnet durch die folgenden Mengen von Puffer B nach den entsprechenden Laufzeiten: |
| | 0 Min. 4,0 % ; 20 Min. 17,0 % ; 30 Min. 37,0 % ; 40 Min. 4,0 % |
| Temperatur: | 35°C |
| Aufgabevolumen: | 10 µl |
| Gesamtlaufzeit: | 55 Min. |
| Flußrate: | 1,0 ml/Min. |
| Detektion: | 214 nm (Waters 2487) |

Zur Ermittlung des Gehalts an Preproinsulin in der Aufgabelösung wurde der Quotient aus der Peakfläche des Preproinsulins in der Analysenprobe und der entsprechenden Peakfläche einer Standardsubstanz gebildet. Für die Ermittlung des Reinheitsgrades wurde der Quotient aus der Peakfläche des Preproinsulins und der Summe der Peakflächen von allen eluierbaren Substanzen in der Analysenprobe gebildet.

### HPLC-GPC-Analyse

| | |
|---|---|
| Säule: | 2 Säulen in Serie, Edelstahl L = 300 mm; ID = 7,8 mm |
| Instrument: | Pumpe: Waters 510 / Autosampler: Wisp 717 |
| Software: | Waters Millenium |
| Stationäre Phase: | Shodex Protein KW 802.5 120-7-Diol |
| Trenngrenzen: | 2 000 bis 80 000 Dalton |
| Mobile Phase: | 30 Vol-% Acetonitril, 3,5 M Essigsäure, pH 3,0 eingestellt mit Ammoniakwasser |
| Gradient: | isocratic |
| Temperatur: | Raumtemperatur |
| Aufgabevolumen: | 100 µl |
| Gesamtlaufzeit: | 65 Min. |
| Flußrate: | 0,5 ml/Min |
| Detektion: | 276 nm (Waters 2487) |

Zur Ermittlung des Anteils an höhermolekularen Substanzen wurde der Quotient aus den Peakflächen aller höhermolekularen Substanzen, die zeitlich vor dem monomeren Preproinsulin eluiert wurden, und der Summe der Peakflächen von allen eluierbaren Substanzen gebildet. Die Retentionszeit für das monomere. Preproinsulin wurde mit einer Standardsubstanz ermittelt.

### Beispiel 1

Aus dem entsprechend gentechnisch modifizierten E.-Coli-Zellen wurde nach Abschluß der oben erwähnten Prozessstufen a, b, c und d eine Lösung des Preproinsulins mit folgender Aminosäuresequenz erhalten:

Dieses Preproinsulin entspricht der Formel 1, dabei ist
- X: eine Peptidkette mit 35 Aminosäureresten mit der Sequenz des C-Peptids vom Affen
- R1: eine Peptidkette mit 10 Aminosäureresten mit der Sequenz: Ala-Thr-Thr-Ser-Thr-Gly-Asn-Ser-Ala-Arg (SEQ ID NO: 5)
- R2: der Aminosäurerest Asn (identisch mit A21 der A-Kette vom Humaninsulin)
- A1-A20: Peptidkette mit der Sequenz (nur A1 bis A20) der A-Kette vom Humaninsulin
- B1-B30: Peptidkette mit der Sequenz der B-Kette vom Humaninsulin

Zur Reinigung der Preproinsulin-Lösung wurde eine Apparatur verwendet, die hauptsächlich aus zwei in Reihe angeordneten Chromatographie-Säulen und einem dazwischen angeordneten Rührgefäß bestand. In dem Rührgefäß wurde inline der pH-Wert der Lösung zwischen den beiden Säulen umgestellt.

In der ersten Chromatograhie-Säule (Hersteller: Pharmacia, Durchmesser: 5 cm) wurde ein Gelbett (Betthöhe: 14 cm, Bettvolumen: 275 ml) mit dem Anionenaustauscherharz DEAE-Sepharose fast flow (Hersteller: Pharmacia Biotech; Prod.-Nr. 17-0709-05) hergestellt. Die Säule wurde von oben nach unten und bei Normaldruck von 1 bar betrieben. Die Flussrate betrug 2000 ml/h. Vor der Säule waren ein Mehrwegeventil, eine Aufgabepumpe (Ismatec MV) und eine Blasenfalle installiert. Über das Mehrwegventil wurden nacheinander folgende Lösungen:

| | |
|---|---|
| 8,1 L | Aufgabelösung |
| 2,3 L | Verdrängungspuffer |
| 1,4 L | Waschpuffer |
| 1,4 L | Regenerationslösung |
| 2 L | Equilibrierpuffer |

auf die Säule gepumpt. Nach der Säule war eine UV-Sonde (275 nm mit Messwertregistrierung) und ein weiteres Mehrwegeventil installiert. Über das zweite Mehrwegventil wurden ca. 10,2 L Permeatfraktion in das oben erwähnte Rührgefäß und anschließend ca. 1 L Waschfraktion in ein Sammelgefäß geleitet. Die übrigen Permeate wurden über das Mehrwegeventil in den Biokanal entsorgt.
Die Chromatographie an dem Anionenaustauscher wurde im Durchflussmodus betrieben, d.h. die Bedingungen (pH 8,3 ; LF = 6,1 mS/cm) wurden so gewählt, dass der Wertstoff Preproinsulin nicht an das Gel gebunden, sondern während der Produktaufgabe mit dem Permeat durch die Säule gespült wurde. Dagegen wurden Verunreinigungen an das Gel adsorbiert und mit dem Waschpuffer und der Regenerierlösung ausgeschleust.

Die verwendeten Lösungen hatten folgende Zusammensetzung:

| Aufgabelösung für Säule 1: | | | | | |
|---|---|---|---|---|---|
| Ausgangslösung | | 8,0 | L | | |
| (Zentrifugenüberstand) | | | | | |
| Natriumchlorid-Lösung 25 %ig | | 100 | ml | | ml/L |
| (w/w) | | | | 12,5 | |
| Natronlauge 10 %ig (w/w) | ca. | 4,5 | ml | | ml/L |
| | | | | 0,6 | |
| | pH | 8,3 | | | |
| Leitfähigkeit | LF | 6,1 | mS/c | | |
| | | | m | | |
| Temperatur | ca. | 5 | °C | | |

| | | | |
|---|---|---|---|
| Gereinigtes Wasser. | | 1 | L |
| Tris-(hydroxymethyl)- | | 4,0 | g/L |
| aminomethan | | | |
| Natriumchlorid | | 2,5 | g/L |
| Salzsäure 25 %ig (w/w) | ca. | 2,5 | ml/L |
| | pH | 8,0 | |
| Leitfähigkeit | ca. | 5,7 | mS/cm |
| Temperatur | | Raumtemperatur | |

| | | | |
|---|---|---|---|
| Gereinigtes Wasser | | 1 | L |
| Tris-(hydroxymethyl)- | | 5,0 | g/L |
| aminomethan | | | |
| Natriumchlorid | | 15 | g/L |
| Salzsäure 25 %ig (w/w) | ca. | 3 | ml/L |
| | pH | 8,0 | |
| Leitfähigkeit | ca. | 24 | mS/cm |
| Temperatur | | Raumtemperatur | |

| | | | | |
|---|---|---|---|---|
| Regenerationslösung für Säule 1 und 2: | | | | |
| Gereingtes Wasser | 0,91 | L | | |
| Natriumchlorid | 40 | g | 40 | g/L |
| | | | | |
| Natronlauge 33 %ig (w/w) | 0,09 | L | 1 | mol/L |

| Equilibrierpuffer für Säule 1: | | | |
|---|---|---|---|
| Gereinigtes Wasser | | 1 | L |
| Tris-(hydroxymethyl)-aminomethan | | 5,0 | g/L |
| Natriumchlorid | | 2,0 | g/L |
| Salzsäure 25 %ig (w/w) | ca. | 3 | ml/L |
| | pH | 8,0 | |
| Leitfähigkeit | ca. | 5,1 | mS/c m |
| Temperatur | | | |
| | | Raumtemperatur | |

Im Säulenauslauf wurde die Permeatfraktion, die den Wertstoff Preproinsulin enthielt, und die Waschfraktion, die den größten Teil der höhermolekularen Verunreinigungen enthielt, aufgefangen:

| | |
|---|---|
| 1. ca. 10,2 L | Permeatfraktion (mit Beginn der Aufgabelösung, ab UV-Wert 20 % |
| aufsteigend | |
| | bis UV-Wert 35 % absteigend, während der Produkt-Verdrängung) |
| 2. ca. 1 L | Waschfraktion |
| | (während der Aufgabe des Waschpuffers, ab UV-Wert 30 % aufsteigend bis UV-Wert 40 % absteigend) |

Alle übrigen Permeate wurden in den Biokanal entsorgt.

Das im Auslauf der Säule 1 gemessene UV-Diagramm ist in Figure 1 gezeigt.

Die Permeatfraktion der ersten Säule wurde in dem Zwischengefäß (Nennvolumen: 4 L, mit Rührer, pH-Sonde und Einleitungsrohr) inline mit 90 %iger Milchsäure auf pH 3,5 eingestellt und anschließend direkt auf die zweite Chromatographie-Säule gepumpt.

In der zweiten Chromatographie-Säule (Hersteller: Pharmacia, Durchmesser: 5 cm) wurde ein Gelbett (Betthöhe: 10,5 cm, Bettvolumen: 206 ml) mit dem Kationenaustauscher Source 30 S (Hersteller: Pharmacia Biotech; Prod.-Nr.: 17-1273-04) hergestellt. Die Säule wurde von oben nach unten und bei Normaldruck von 1 bar betrieben. Die Flussrate betrug ebenfalls 2000 ml/h. Vor der Säule war ein Mehrwegeventil, eine Aufgabepumpe und eine Blasenfalle installiert. Über das Mehrwegeventil wurden nacheinander folgende Lösungen:

| | |
|---|---|
| 10,2 L | Aufgabelösung (= Permeatfraktion der Säule 1, auf pH 3,5 eingestellt) |
| 0,5 L | Verdrängungspuffer |
| 3,0 L | Elutionspuffer A/B (aus gleichen Teilen A und B) |
| 2,3 L | Regenerationslösung |
| 2 L | Equilibrierpuffer |

auf die Säule gepumpt. Nach der Säule war eine UV-Sonde (275 nm mit Messwertregistrierung) und ein weiteres Mehrwegeventil installiert. Über das zweite Mehrwegeventil wurden ca. 1 L Hauptfraktion in ein Sammelgefäß geleitet. Die übrigen Permeate wurde über das Mehrwegventil in den Biokanal entsorgt.

Die Chromatographie an dem Kationenaustauscher wurde im Adsorptionsmodus betrieben, d.h. der Wertstoff Preproinsulin wurde während der Produktaufgabe an das Gel adsorbiert und (nach dem Verdrängen der Aufgabelösung) mit dem Elutionspuffer A/B wieder desorbiert. Um einen optimalen Reinigungseffekt zu erzielen, wurde ein linear ansteigender Natriumchlorid-Gradient im Elutionspuffer angewendet.

Die verwendeten Lösungen hatten folgende Zusammensetzung:

| Aufgabelösung für Säule 2: | | | | | |
|---|---|---|---|---|---|
| Permeatfraktion der Säule | ca. | 10,2 | L | | |
| 1 | | | | | |
| Milchsäure 90 %ig | | 14,3 | ml | | ml/L |
| | | | | 1,4 | |
| | pH | 3,5 | | | |
| Leitfähigkeit | ca. | 6,3 | mS/cm | | |
| Temperatur | ca. | 5 | °C | | |

| Verdrängungspuffer für Säule 2: | | | | | |
|---|---|---|---|---|---|
| Gereinigtes Wasser | | 1 | L | | |
| Milchsäure 90 %ig | | 8,3 | ml | | mol/L |
| | | | | 0,1 | |
| Natriumchlorid | | 2,5 | g | | g/L |
| | | | | 2,5 | |
| Natronlauge 10 %ig (w/w) | ca. | 8 | ml | | |
| | pH | 3,5 | | | |
| Leitfähigkeit | ca. | 8 | mS/c | | |
| | | m | | | |
| Temperatur | | Raumtemperatur | | | |

Elutionspuffer A für Säule 2:

Der Elutionspuffer A ist mit dem Verdrängungspuffer für die Säule 2 identisch.

| Elutionspuffer B für Säule 2: | | | | | |
|---|---|---|---|---|---|
| Gereinigtes Wasser | | 1 | L | | |
| Milchsäure 90 %ig | | 8,3 | ml | | mol/L |
| | | | | 0,1 | |
| Natriumchlorid | | 15,0 | g | | g/L |
| | | | | 15,0 | |
| Natronlauge 10 %ig (w/w) | ca. | 7 | ml | | |
| | pH | 3,5 | | | |
| Leitfähigkeit | ca. | 25 | mS/c | | |
| | | | m | | |
| Temperatur | | Raumtemperatur | | | |

| Regenerationslösung für Säule 1 und 2: | | | | |
|---|---|---|---|---|
| Gereinigtes Wasser | 0,91 | L | | |
| Natriumchlorid | 40 | g | 40 | g/L |
| Natronlauge 33 %ig (w/w) | 0,09 | L | 1 | mol/L |

| Equilibrierpuffer für Säule 2: | | | |
|---|---|---|---|
| Gereinigtes Wasser | 1 | L | |
| Milchsäure 90 %ig | 8,3 | g | 0,1 mol/L |
| Natriumchlorid | 2,9 | g/L | |
| Natronlauge 10 %ig (w/w) | ca. | 9 | ml |
| | pH | 3,5 | |
| Leitfähigkeit | ca. | 8,5 | mS/cm |
| Temperatur | | Raumtemperatur | |

Im Säulenauslauf wurden die Hauptfraktion, die den Wertstoff Preproinsulin enthielt, aufgefangen:

| | | |
|---|---|---|
| ca. | 1,0 L | Hauptfraktion |
| | | (während der Elution, ab UV-Wert 65 % aufsteigend bis UV-Wert 76 % absteigend) |

Alle übrigen Permeate wurden in den Biokanal verworfen.

Das im Auslauf der Säule 2 gemessene UV-Diagramm ist in Figure 2 gezeigt.

In der gereinigten Lösung (Hauptfraktion der Säule 2) wurden 15 g/L Preproinsulin mit einem Reinheitsgrad von 89 Flächen-% gemessen (HPLC-RP-Analyse). Die Ausbeute betrug 91 % bezogen auf die Menge an Preproinsulin in der Ausgangslösung. Mit der HPLC-GPC-Analyse wurden 0,2 Flächen-% höhermolekulare Anteile bestimmt.

### Beispiel 2

Aus dem entsprechend gentechnisch modifizierten E.-Coli-Zellen wird nach Abschluß der oben erwähnten Prozessstufen a, b, c und d eine Lösung des Preproinsulins mit folgender Aminosäuresequenz erhalten:

Dieses Preproinsulin entspricht der Formel 1, dabei ist

| | |
|---|---|
| X | eine Peptidkette mit 35 Aminosäureresten mit der Sequenz des C-Peptids vom Affen |
| R1 | eine Peptidkette mit 10 Aminosäureresten mit der Sequenz: Ala-Thr-Thr-Ser-Thr-Gly-Asn-Ser-Ala-Arg (SEQ ID NO: 5) |
| R2 | der Aminosäurerest Gly |
| A1-A20 | Peptidkette mit der Sequenz (nur A1 bis A20) der A-Kette vom Humaninsulin |
| B1-B30 | Peptidkette mit der Sequenz der B-Kette vom Humaninsulin |

Zur Reinigung der Preproinsulin-Lösung wurde wieder eine Apparatur verwendet, die hauptsächlich aus zwei in Reihe angeordneten Chromatographie-Säulen und einem dazwischen angeordneten Rührgefäß bestand. In dem Rührgefäß wurde inline der pH-Wert der Lösung zwischen den beiden Säulen umgestellt. Die Apparate für die zweite Chromatographie-Stufe waren druckstabil ausgelegt.

Die Chromatographie auf der Säule 1 und die Umstellung des pH-Wertes in dem Zwischengefäß wurde wie im Beispiel 1 beschrieben durchgeführt, so dass die Beschreibung und die Werte hier nicht noch einmal genannt werden.

In der zweiten Chromatographie-Säule (Hersteller: Prochrom, Durchmesser: 5 cm, Material: Edelstahl) wurde ein Gelbett (Betthöhe: 10 cm, Bettvolumen: 196 ml) mit dem Kationenaustauscher Source 30 S (Hersteller: Pharmacia Biotech; Prod.-Nr.: 17-1273-04) hergestellt. Die Säule wurde von oben nach unten und bei einem Arbeitsdruck von 10 bar betrieben. Die Flussrate betrug 3500 ml/h. Vor der Säule war ein Mehrwegeventil, eine Aufgabepumpe (Hersteller: Besta; Typ: HD2-300) installiert. Über das Mehrwegeventil wurden nacheinander folgende Lösungen:

| | |
|---|---|
| 10,2 L | Aufgabelösung (= Permeatfraktion der Säule 1, auf pH 4,6 eingestellt) |
| 0,5 L | Verdrängungspuffer |
| 3,0 L | Elutionspuffer A/B (aus gleichen Teilen A und B) |
| 2,3 L | Regenerationslösung |
| 2 L | Equilibrierpuffer |

auf die Säule gepumpt. Nach der Säule war eine UV-Sonde (275 nm mit Messwertregistrierung) und ein weiteres Mehrwegeventil installiert. Über das zweite Mehrwegeventil wurde die Hauptfraktion, die das gereinigte Preproinsulin enthielt, in ein Sammelgefäß geleitet. Die übrigen Permeate wurden über das Mehrwegventil in den Biokanal entsorgt.

Die Chromatographie an dem Kationenaustauscher wurde im Adsorptionsmodus betrieben, d.h. der Wertstoff Preproinsulin wurde während der Produktaufgabe an das Gel adsorbiert und (nach dem Verdrängen der Aufgabelösung) mit dem Elutionspuffer A/B wieder desorbiert. Um einen optimalen Reinigungseffekt zu erzielen, wurde ein linearer Natriumchlorid-Gradient im Elutionspuffer angewendet.

Die verwendeten Lösungen hatten folgende Zusammensetzung:

| Aufgabelösung für Säule 2: | | | | | |
|---|---|---|---|---|---|
| Permeatfraktion der Säule 1 | ca. | 10,2 | L | | |
| Milchsäure 90 %ig | | 12,2 | ml | 1,2 | ml/L |
| | pH | 4,6 | | | |
| Leitfähigkeit | ca. | 6,7 | mS/c | | |
| | | | m | | |
| Temperatur | ca. | 5 | °C | | |

| Verdrängungspuffer für Säule 2: | | | | | |
|---|---|---|---|---|---|
| Gereinigtes Wasser | | 1 | L | | |
| Milchsäure 90 %ig | | 8,3 | ml | | mol/L |
| | | | | 0,1 | |
| Natriumchlorid | | 2,5 | g | | g/L |
| | | | | 2,5 | |
| Natronlauge 10 %ig (w/w) | ca. | 27 | ml | | |
| | pH | 4,6 | | | |
| Leitfähigkeit | ca. | 8 | mS/cm | | |
| Temperatur | | | | | |
| | | Raumtemperatur | | | |

Elutionspuffer A für Säule 2:
Der Elutionspuffer A ist mit dem Verdrängungspuffer für die Säule 2 identisch.

| Elutionspuffer B für Säule 2: | | | | | |
|---|---|---|---|---|---|
| Gereinigtes Wasser | | 1 | L | | |
| Milchsäure 90 %ig | | 8,3 | ml | | mol/L |
| | | | | 0,1 | |
| Natriumchlorid | | 15,0 | g | | g/L |
| | | | | 15,0 | |
| Natronlauge 10 %ig (w/w) | ca. | 27 | ml | | |
| | pH | 4,6 | | | |
| Leitfähigkeit | ca. | 25 | mS/cm | | |
| Temperatur | | | | | |
| | | Raumtemperatur | | | |

| Regenerationslösung für Säule 1 und 2: | | | | | |
|---|---|---|---|---|---|
| Gereinigtes Wasser | | 0,91 | L | | |
| Natriumchlorid | | 40 | g | 40 | g/L |
| Natronlauge 33 %ig (w/w) | | 0,09 | L | 1 | mol/L |

| Equilibrierpuffer für Säule 2: | | | | | |
|---|---|---|---|---|---|
| Gereinigtes Wasser | | 1 | L | | |
| Milchsäure 90 %ig | | 8,3 | g | | mol/L |
| | | | | 0,1 | |
| Natriumchlorid | | 2,9 | g/L | | |
| Natronlauge 10 %ig (w/w) | ca. | 26 | ml | | |
| | pH | 4,6 | | | |
| Leitfähigkeit | ca. | 8,7 | mS/cm | | |
| Temperatur | | | | | |
| | | Raumtemperatur | | | |

Im Säulenauslauf wurden die Hauptfraktion, die den Wertstoff Preproinsulin enthielt, aufgefangen:

| | | | |
|---|---|---|---|
| ca. | 0,9 | L | Hauptfraktion |
| | | | (während der Elution, ab UV-Wert 65 % aufsteigend bis UV-Wert 76 % absteigend) |

Alle übrigen Permeate wurden in den Biokanal verworfen.
In der gereinigten Lösung (Hauptfraktion der Säule 2) wurden 17 g/L Preproinsulin mit einem Reinheitsgrad von 93 Flächen-% gemessen (HPLC-RP-Analyse). Die Ausbeute betrug 92 % bezogen auf die Menge an Preproinsulin in der Ausgangslösung. Mit der HPLC-GPC-Analyse wurden <0,1 Flächen-% höhermolekulare Anteile bestimmt.

### Beispiel 3

Aus dem entsprechend gentechnisch modifizierten E.-Coli-Zellen wird nach Abschluß der oben erwähnten Prozessstufen a, b, c und d eine Lösung des Preproinsulins mit folgender Aminosäuresequenz erhalten:

Dieses Preproinsulin entspricht der Formel 1, dabei ist
- X: eine Peptidkette mit 29 Aminosäureresten mit der Sequenz:
- R1: eine Peptidkette mit 10 Aminosäureresten mit der Sequenz: Ala-Thr-Thr-Ser-Thr-Gly-Asn-Ser-Ala-Arg (SEQ ID NO: 5)
- R2: der Aminosäurerest Asn (A21 der A-Kette vom Humaninsulin)
- A1-A20: Peptidkette mit der Sequenz (nur A1 bis A20) der A-Kette vom Humaninsulin B1-B30Peptidkette mit einer analogen Sequenz zur B-Kette vom Humaninsulin, d.h. mit einem Austausch von Lys gegen Val in der Position B3 und einem Austausch von Glu gegen Lys in Position B29.

Zur Reinigung der Preproinsulin-Lösung wurde die gleiche Apparatur verwendet, die im Beispiel 1 verwendet wurde.

Für die Chromatographie auf der Säule 1 wurde diesmal das Anionenaustauscherharz Source 30 Q (Hersteller: Pharmacia Biotech; Prod.-Nr.: 17-1275-04) verwendet. Zur Regeneration dieses Gels wurde die doppelte Menge an Regenerationslösung im Vergleich zu den Beispielen 1 und 2 gebraucht. Die übrigen Parameter der ersten Chromatographie, wie Zusammensetzung und Volumina der Lösungen, waren die gleichen wie in den Beispielen 1 und 2 beschrieben.

Ebenso wurde die Umstellung des pH-Wertes in dem Zwischengefäß wie im Beispiel 1 beschrieben durchgeführt.

Die zweite Chromatographie wurde diesmal bei einem Arbeitsdruck von 15 bar durchgeführt. Alle übrigen Parameter der zweiten Chromatographie waren die gleichen wie im Beispiele 2 beschrieben.

In der gereinigten Lösung (Hauptfraktion der Säule 2) wurden 17 g/L Preproinsulin mit einem Reinheitsgrad von 92,5 Flächen-% gemessen (HPLC-RP-Analyse). Die Ausbeute betrug 91 % bezogen auf die Menge an Preproinsulin in der Ausgangslösung. Mit der HPLC-GPC-Analyse wurden <0,1 Flächen-% höhermolekulare Anteile bestimmt.

### Denaturierungstest

Mit dem Denaturierungstest (Tabelle 1) wird gezeigt, dass die höhermolekularen polymeren Formen der Preproinsuline, wie sie während der Faltungsreaktion entstehen, die Denaturierung von nativem Insulin induzieren können.

In dem Denaturierungstest wurde natives Insulin glargin, ein Produkt der Firma Aventis Deutschland GmbH, das nach der enzymatischen Spaltung des im Beispiel 2 beschriebenen Preproinsulins erhalten wird, kristallisiert. Überraschenderweise konnten wir in eigenen Versuchen zeigen, dass unter den Bedingungen der Kristallisation des Insulin glargin (pH 6,1 und 26 °C), es zu einer Denaturierung des nativen Insulins kommt, wenn dem Kristallisieransatz Substanzen zugesetzt werden, die die Denaturierung von Insulin induzieren können.

Für die Kristallisieransätze wurde eine Standard-Lösung folgender Zusammensetzung hergestellt:

| | | |
|---|---|---|
| insulin glargin | 5 | g/L |
| Zitronensäure | 5,2 | mmole/L |
| Zinkchlorid | 3 | mmole/L |
| Natriumchlorid | 0,5 | g/L |
| n-Propanol | 7 | %(v/v) |
| gereinigtes Wasser ad | 500 | ml |
| mit 1 N Salzsäure pH | 3 | |

Die Lösung wurde durch ein Membranfilter mit 0,1 µm Porenweite filtriert.

In dem Denaturierungstest wurden zu dieser sauren Standardlösung Lösungen die verschiedenen Testsubstanzen zugesetzt: Die Waschfraktion der Säule 1, die die abgetrennten polymeren Formen der Preproinsuline in einer Konzentration von 5 g/L enthielt oder die Hauptfraktion der Säule 2, die das gereinigte Preproinsulin in einer Konzentration von 15 bzw. 17 g/L enthielt. Als weiterer Beweis, dass die beobachteten Phänomene durch die Denaturierung von Insulin hervorgerufen wurden, diente der Zusatz von 10 ml einer 0,1 %igen wässrigen Stammlösung von Poloxamer 171. Es ist bekannt, dass Poloxamer 171 die Denaturierung von Insulin an hydrophoben Grenzflächen unterdrücken kann (H.Thurow and K.Geisen, Diabetologia (1984) 27, 212-218 and EP 0 018 609).

Anschließend wurden die Lösungen auf 26 °C temperiert und unter Rühren mit 10 %iger Natronlauge auf pH 6,1 eingestellt, wobei das Insulin amorph ausfiel. Die amorphe Suspension wurde bei 26 °C über 50 Stunden gerührt. Nach dieser Zeit waren in allen Ansätzen Insulinkristalle entstanden.

Zur Auswertung der Ansätze wurden Proben unter dem Mikroskop beurteilt, wobei auf das Auftreten von amorphen Partikeln (Schleier) im Hintergrund bzw. zwischen den Insulinkristallen geachtet wurde. Zusätzlich wurde jeder Ansatz in 2 etwa gleichgroße Teile geteilt. Der 1. Teil wurde zur Untersuchung des Sedimentationsverhaltens in ein 250 ml - Messzylinder gegeben, wobei nach 60 Min. Stehen bei Raumtemperatur das Volumen des Sediments und die Klarheit des Überstandes bewertet wurden. Der zweite Teil wurde mit 1 N Salzsäure auf pH 3 eingestellt, wobei nach Auflösen der Insulinkristalle die Klarheit der entstandenen Lösung bewertet wurde.

Das Ergebnis des Denaturierungstests ist in Tabelle 1 gezeigt. In den Kontrollproben 174 A und 188 A ohne Zusatz der Polymerfraktion war keine Denaturierung beobachtet worden. Im Mikroskop waren Kristalle vor einem klaren Hintergrund zu erkennen. Nach 60 Minuten war die Kristalle sedimentiert, wobei eine kompakten Sediment und ein klarer Überstand entstanden waren. Nach Auflösen der Kristalle bei pH 3 war eine klare Lösung entstanden. Dagegen wurde in den Proben 174 B, 188 B bzw. 174 C, bei denen zum Kristallisieransatz 1 ml bzw. 5 ml Polymerfraktion zugesetzt worden waren, eine deutliche Denaturierung von Insulin glargin beobachtet. Im Mikroskop war zwischen den Kristallen ein amorpher Schleier zu erkennen. Im Sedimentationstest waren voluminöse Sedimente mit einem Sedimentvolumen von 50 bis 90 ml (aus 250 ml Kristallsuspension) entstanden. Nach Wiederauflösen der Kristalle bei pH 3 waren mehr oder weniger trübe amorphe Suspensionen entstanden. In Gegenwart von 20 ppm Poloxamer 171 war bei einem Zusatz von 1 ml Polymerlösung (188 C) keine Denaturierung beobachtet worden. In den Testansätzen 174 D, 174 E und 174 F, denen gereinigte Preproinsuline (Hauptfraktion der Säule 2 aus den Beispielen 1, 2 und 3) zugesetzt worden waren, konnte ebenfalls keine Denaturierung von Insulin glargin beobachtet werden.

Ähnliche, hier nicht gezeigte Ergebnisse wurden in einem analogen Denaturierungstest erhalten, bei dem Humaninsulin kristallisiert wurde.

| Tabelle 1 | | | | |
|---|---|---|---|---|
| Einfluß der höhermolekularen Verunreinigungen auf die Denaturierung von Insulin Glargin | | | | |
| **Kristallisieransatz Nr.** | **Zusätze zum Kristallisationsansatz** | **Mikroskopisches Bild Aussehen des Hintergrundes (zwischen den Rhomboeder-Kristallen)** | **Aussehen nach Lösen der Kristalle bei pH 3** | **Sedimentationsverhalten der Kristallsuspension** |
| 174 A | Keine | klar | klar | + + + |
| 174 B | 1 ml Waschfrkt. Säule 1 * | Amorphe Partikel | schwach trüb | + - - |
| 174 C | 5 ml Waschfrkt. Säule 1 * | stärker amorphe Partikel | trüb | - - - |
| | | | | |
| 174 D | 1 ml Hauptfrkt. Beispiel 1 ** | klar | klar | + + + |
| 174 E | 1 ml Hauptfrkt. Beispiel 2 *** | klar | klar | + + + |
| 174 F | 1 ml Hauptfrkt. Beispiel 3 **** | klar | klar | + + + |
| | | | | |
| 188 A | Keine | klar | klar | + + + |
| 188 B | 1 ml Waschfrkt. Säule 1 * | Amorphe Partikel | trüb | - - - |
| 188 C | 1 ml Waschfrkt. Säule 1 * und 20 ppm Poloxamer 171 | vereinzelt amorphe Partikel | klar | + + + |

| | | | | |
|---|---|---|---|---|
| Kristallisieransätze von jeweils 500 ml mit 2500 mg Insulin Glargin (= 5 g/L). * Die Waschfraktion der Säule 1 aus Beispiel 2 enthielt 5 g/L Protein. + + + = Nach ca. 60 Min. ein Sediment von 3 bis 5 ml aus 250 ml Suspension. ** Die Hauptfraktion der Säule 2 aus Beispiel 1 enthielt 15 g/L Preproinsulin - - - = Nach ca. 60 Min. ein Sediment bis zu 90 ml aus 250 ml Suspension. *** Die Hauptfraktion der Säule 2 aus Beispiel 2 enthielt 17 g/L Preproinsulin **** Die Hauptfraktion der Säule 2 aus Beispiel 3 enthielt 17 g/ | | | | |

## Patentansprüche

1. Verfahren zur chromatographischen Reinigung von Preproinsulin der Formel 1, 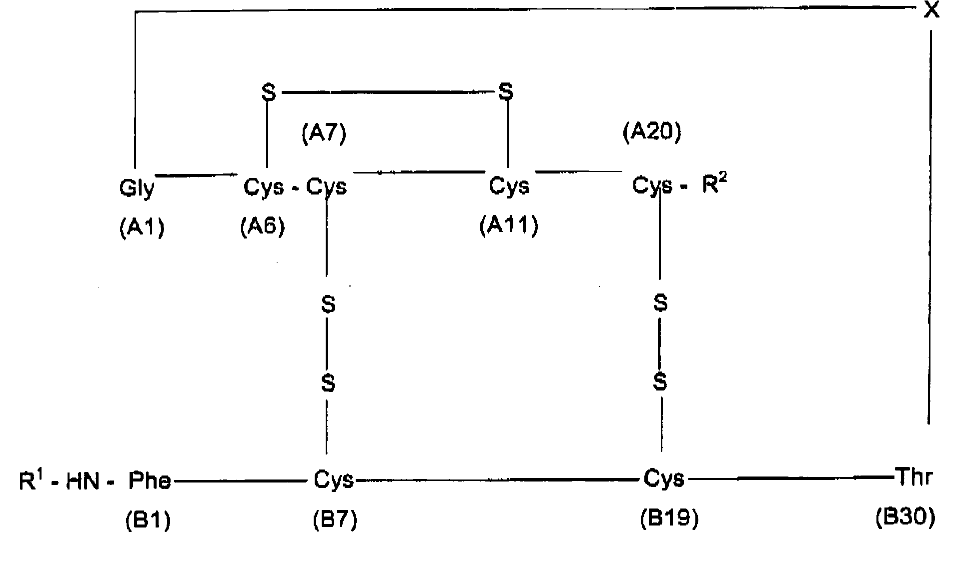
R¹-B¹-B30-X-A1-A20-R² (1)
in der
X a) ein genetisch kodierbarer Aminosäurerest oder
b) ein Peptid mit 2 bis 35 Aminosäureresten, wobei am Anfang und am Ende des Peptids jeweils ein basischer Aminosäurerest, insbesondere Arg steht, und für den Fall, dass das Peptid aus mehr als 3 Aminosäureresten besteht, am Anfang und Ende des Peptids jeweils zwei basische Aminosäurereste, insbesondere Arg und/oder Lys stehen,
R¹ a) ein Wasserstoffatom,
b) ein genetisch kodierbarer Aminosäurerest oder
c) ein Peptid mit 2 bis 15 Aminosäureresten,
R² ein genetisch kodierbarer Aminosäurerest ist und
und die Reste A1 - A20 der Aminosäuresequenz der A-Kette von Humaninsulin oder einem Insulinanalogon entsprechen und die Reste B1 - B30 der Aminosäuresequenz der B-Kette von Humaninsulin oder einem Insulinanalogon entsprechen;
bei dem höhermolekulare Substanzen aus einer wäßrigen Lösung des Preproinsulins durch eine erste Chromatographie an einem Anionenaustauscher Im Durchflussmodus und eine anschließende zweite Chromatographie an einem Kationenaustauscher im Adsorptionsmodus abgetrennt werden.

2. Verfahren nach Anspruch 1, wobei das Preproinsulin folgende Aminosäuresequenz hat:

3. Verfahren nach Anspruch 1, wobei das Preproinsulin folgende Aminosäuresequenz hat:

4. Verfahren nach Anspruch 1, wobei das Preproinsulin folgende Aminosäuresequenz hat:

5. Verfahren gemäss den Ansprüchen 1 bis 4 zur Abtrennung von Fremdsubstanzen aus den Lösungen von Preproinsulinen, welche die Denaturierung von Insulin induzieren.

6. Verfahren gemäss den Ansprüchen 1 bis 5, **dadurch gekennzeichnet, dass** die zweite Chromatographie bei einem pH-Wert von 3,0 bis 5,5 durchgeführt wird.

7. Verfahren gemäss den Ansprüchen 1 bis 6, **dadurch gekennzeichnet, dass** die zweite Chromatographie bei einem Druck von 1 bis 30 bar durchgeführt wird.

8. Verfahren zur Herstellung von Insulin durch Expression ungefalteten Preproinsulins, umfassend die Schritte:
a) Fermentation von gentechnisch veränderten Mikroorganismen, welche ungefaltetes Preproinsulin exprimieren
b) Ernten der Mikrooganismen und Zellaufschluß
c) Isolierung der Einschlußkörper mit ungelöstem, ungefalteten Preproinsulin
d) Auflösen des Preproinsulins mit korrekter Faltung der Peptidkette und mit gleichzeitiger Schließung der Disulfidbrücken zum Preproinsulin und anschließendem Durchlaufen eines Verfahrens gemäß den Ansprüchen 1 - 7
e) Enzymatische Spaltung des Preproinsulins zum Humaninsulin
f) Reinigung des Humaninsulins
g) Kristallisation des Humaninsulins und Trocknung

## Claims

1. A method for the chromatographic purification of preproinsulin of the formula 1, 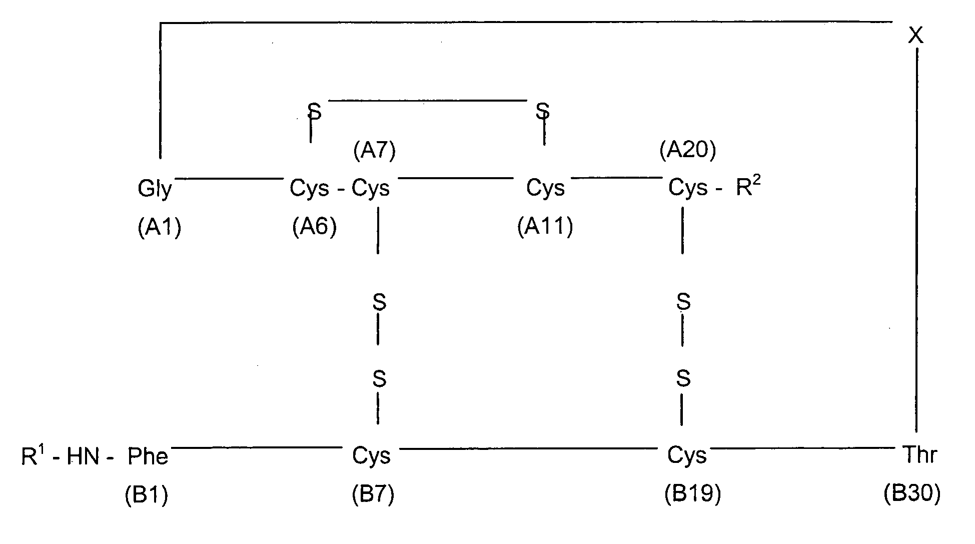
R¹-B1-B30-X-A1-A20-R² (1)
in which
X a) is a genetically encodable amino acid residue or
b) is a peptide having from 2 to 35 amino acid residues, which starts and ends with in each case a basic amino acid residue, in particular Arg, and which, if it consists of more than 3 amino acid residues, starts and ends with in each case two basic amino acid residues, in particular Arg and/or Lys,
R¹ a) is a hydrogen atom,
b) is a genetically encodable amino acid residue or
c) is a peptide having from 2 to 15 amino acid residues,
R² is a genetically encodable amino acid residue, and
the residues A1 - A20 correspond to the amino acid sequence of the A chain of human insulin or of an insulin analog and the residues B1 - B30 correspond to the amino acid sequence of the B chain of human insulin or of an insulin analog;
in which method higher molecular weight substances are removed from an aqueous solution of said preproinsulin by means of a first chromatography on an anion exchanger in flow-through mode and a subsequent second chromatography on a cation exchanger in adsorption mode.

2. The method as claimed in Claim 1, wherein said preproinsulin has the following amino acid sequence:

3. The method as claimed in Claim 1, wherein said preproinsulin has the following amino acid sequence:

4. The method as claimed in Claim 1, wherein said preproinsulin has the following amino acid sequence:

5. The method as claimed in any of claims 1 to 4 for separating foreign substances from the solutions of preproinsulins which induce insulin denaturation.

6. The method as claimed in any of claims 1 to 5, wherein the second chromatography is carried out at a pH of from 3.0 to 5.5.

7. The method as claimed in any of claims 1 to 6, wherein the second chromatography is carried out under a pressure of from 1 to 30 bar.

8. A method for preparing insulin by expressing nonfolded preproinsulin, comprising the steps:
a) fermentation of genetically modified microorganisms which express nonfolded preproinsulin,
b) harvesting the microorganisms and cell disruption,
c) isolating the inclusion bodies containing undissolved; nonfolded preproinsulin,
d) dissolving the preproinsulin with correct folding of the peptide chain and simultaneous closure of the disulfide bridges to give preproinsulin, and subsequently running a method as claimed in claims 1 - 7
e) enzymic cleavage of preproinsulin to give human insulin,
f) purification of human insulin,
g) crystallization of human insulin and drying.

## Revendications

1. Procédé pour la purification chromatographique de la préproinsuline de formule 1 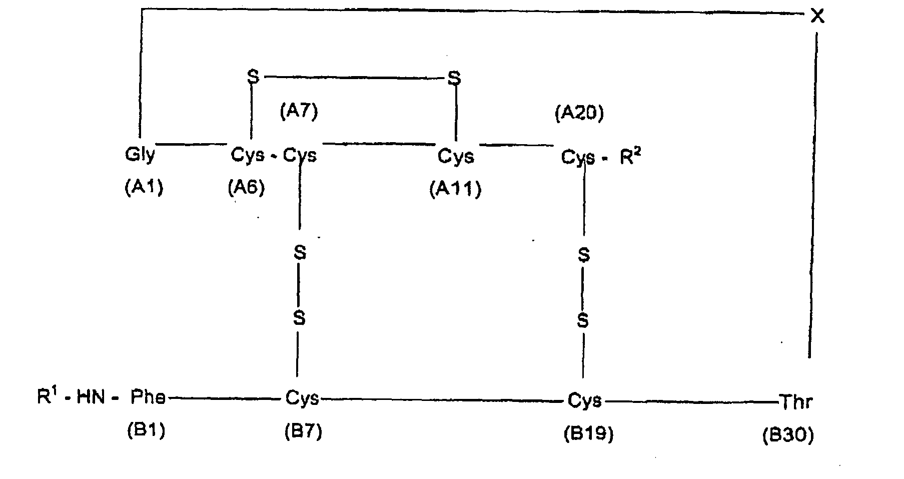
R¹-B1-B30-X-A1-A20-R² (1)
dans laquelle
X est
a) un résidu d'acides aminés pouvant subir un codage génétique, ou
b) un peptide ayant 2 à 35 résidus d'acides aminés, un résidu d'acide aminé basique, en particulier Arg, se trouvant tant au début qu'à l'extrémité du peptide, et, dans le cas dans lequel le peptide est constitué de plus de 3 résidus d'acides aminés, deux résidus d'acides aminés basiques, en particulier Arg et/ou Lys, se trouvant tant au début qu'à l'extrémité du peptide,
R¹ est
a) un atome d'hydrogène,
b) un résidu d'acide aminé pouvant subir un codage génétique, ou
c) un peptide ayant 2 à 15 résidus d'acides aminés,
R² est un résidu d'acide aminé pouvant subir un codage génétique, et
les résidus A1-A20 correspondent à la séquence d'acides aminés de la chaîne A de l'insuline humaine ou d'un analogue de l'insuline, et les résidus B1-B30 correspondent à la séquence d'acides aminés de la chaîne B de l'insuline humaine ou d'un analogue de l'insuline ;
procédé dans lequel des substances à grande masse moléculaire sont séparées d'une solution aqueuse de la préproinsuline par une première chromatographie sur un échangeur d'ions en mode passage direct, et une deuxième chromatographie, effectuée ensuite, sur un échangeur de cations en mode adsorption.

2. Procédé selon la revendication 1, dans lequel la préproinsuline a la séquence d'acides aminés suivante:

3. Procédé selon la revendication 1, dans lequel la préproinsuline a la séquence d'acides aminés suivante :

4. Procédé selon la revendication 1, dans lequel la préproinsuline a la séquence d'acides aminés suivante :

5. Procédé selon les revendications 1 à 4 pour séparer des substances étrangères à partir des solutions de préproinsulines, qui induisent la dénaturation de l'insuline.

6. Procédé selon les revendications 1 à 5, **caractérisé en ce que** la deuxième chromatographie est mise en oeuvre à un pH de 3,0 à 5,5.

7. Procédé selon les revendications 1 à 6, **caractérisé en ce que** la deuxième chromatographie est mise en oeuvre sous une pression de 1 à 30 bar.

8. Procédé de préparation d'insuline par expression de préproinsuline dépliée, comprenant les étapes suivantes :
a) fermentation de microorganismes ayant subi une modification génétique, qui expriment la préproinsuline dépliée,
b) récolte des microorganismes et rupture des cellules,
c) isolement des corps d'inclusion avec de la préproinsuline dépliée non dissoute,
d) dissolution de la préproinsuline, avec repliement correct de la chaîne peptidique, avec simultanément fermeture des ponts disulfure conduisant à la préproinsuline, puis mise en oeuvre d'un procédé selon les revendications 1 à 7,
e) dissociation enzymatique de la préproinsuline, conduisant à l'insuline humaine,
f) purification de l'insuline humaine,
g) cristallisation de l'insuline humaine et séchage.


### SEQUENZPROTOKOLL

<110> Aventis Pharma Deutschland GmbH
<120>
<130>
<160> 4
<170> Patent In Ver. 2.1
<210> 1
   <211> 29
   <212> PRT
   <213> Künstliche Sequenz
<220>
   <223> Beschreibung der künstlichen Sequenz:C-Peptid
<400> 1
   Arg Asp Val Pro Gln Val Glu Leu Gly Gly Gly Pro Gly Ala Gly Ser
   1               5                   10                  15
   Leu Gln Pro Leu Ala Leu Glu Gly Ser Leu Gln Lys Arg
               20                  25
<210> 2
   <211> 96
   <212> PRT
   <213> Künstliche Sequenz
<220>
   <223> Beschreibung der künstlichen Sequenz:Preproinsulin I
<400> 2
   Ala Thr Thr Ser Thr Gly Asn Ser Ala Arg Phe Val Asn Gln His Leu
   1               5                   10                  15
   Cys Gly Ser His Leu Val Glu Ala Leu Tyr Leu Val Cys Gly Glu Arg
               20                  25                  30
   Gly Phe Phe Tyr Thr Pro Lys Thr Arg Arg Glu Ala Glu Asp Pro Gln
           35                  40                  45
   Val Gly Gln Val Glu Leu Gly Gly Gly Pro Gly Ala Gly Ser Leu Gln
       50                  55                  60
   Pro Leu Ala Leu Glu Gly Ser Leu Gln Lys Arg Gly Ile Val Glu Gln
   65                  70                  75                  80
   Cys Cys Thr Ser Ile Cys Ser Leu Tyr Gln Leu Glu Asn Tyr Cys Asn
                   85                  90                  95
<210> 3
   <211> 96
   <212> PRT
   <213> Künstliche Sequenz
<220>
   <223> Beschreibung der künstlichen Sequenz:Preproinsulin II
<400> 3
   Ala Thr Thr Ser Thr Gly Asn Ser Ala Arg Phe Val Asn Gln His Leu
   1               5                   10                  15
   Cys Gly Ser His Leu Val Glu Ala Leu Tyr Leu Val Cys Gly Glu Arg
               20                  25                  30
   Gly Phe Phe Tyr Thr Pro Lys Thr Arg Arg Glu Ala Glu Asp Pro Gln
           35                  40                  45
   Val Gly Gln Val Glu Leu Gly Gly Gly Pro Gly Ala Gly Ser Leu Gln
       50                  55                  60
   Pro Leu Ala Leu Glu Gly Ser Leu Gln Lys Arg Gly Ile Val Glu Gln
   65                  70                  75                  80
   Cys Cys Thr Ser Ile Cys Ser Leu Tyr Gln Leu Glu Asn Tyr Cys Gly
                   85                  90                  95
<210> 4
   <211> 90
   <212> PRT
   <213> Künstliche Sequenz
<220>
   <223> Beschreibung der künstlichen Sequenz:Preproinsulin III
<400> 4
   Ala Thr Thr Ser Thr Gly Asn Ser Ala Arg Phe Val Lys Gln His Leu
    1               5                   10                  15
   Cys Gly Ser His Leu Val Glu Ala Leu Tyr Leu Val Cys Gly Glu Arg
               20                  25                  30
   Gly Phe Phe Tyr Thr Pro Glu Thr Arg Asp Val Pro Gln Val Glu Leu
           35                  40                  45
   Gly Gly Gly Pro Gly Ala Gly Ser Leu Gln Pro Leu Ala Leu Glu Gly
       50                  55                  60
   Ser Leu Gln Lys Arg Gly Ile Val Glu Gln Cys Cys Thr Ser Ile Cys
   65                  70                  75                  80
   Ser Leu Tyr Gln Leu Glu Asn Tyr Cys Asn
                   85                  90